# EUROPEAN PATENT SPECIFICATION

(11) **EP 4 358 984 B1**
(45) Date of publication and mention of the grant of the patent: **17.06.2026**
(21) Application number: 22743938.7
(22) Date of filing: 22.06.2022
(51) Int. Cl.: A61K 35/763, A61P 35/00, A61K 31/716, A61K 39/395, A61K 45/06, C07K 16/28, C12N 7/00

(54) **COMBINATION OF AN ONCOLYTIC VIRUS, AN ANTI-PD-1 INHIBITOR AND AN ACTIVATOR OF THE INNATE IMMUNE SYSTEM FOR USE IN THE TREATMENT OF PROSTATE CANCER**
KOMBINATION AUS EINEM ONKOLYTISCHEN VIRUS, EINEM ANTI-PD-1-HEMMER UND EINEM AKTIVATOR DES ANGEBORENEN IMMUNSYSTEMS ZUR VERWENDUNG BEI DER BEHANDLUNG VON PROSTATAKREBS
COMBINAISON D'UN VIRUS ONCOLYTIQUE, D'UN INHIBITEUR ANTI-PD-1 ET D'UN ACTIVATEUR DU SYSTÈME IMMUNITAIRE INNÉ POUR UNE UTILISATION DANS LE TRAITEMENT DU CANCER DE LA PROSTATE

(30) Priority: 24.06.2021 NO 20210816
(43) Date of publication of application: 01.05.2024
(73) Proprietor: Folkan, Gro, 9015 Tromsø (NO)
(72) Inventor: ROLF, Seljelid, 9015 TROMSØ (NO)
(74) Representative: Zacco Norway AS
(86) International application number: PCT/NO2022/050144
(87) International publication number: WO 2022/271033

(56) References cited:
- WO-A1-2018/045058
- US-A1- 2005 065 114
- US-A1- 2021 008 135
- SELJELID R: "A watersoluble aminated gl-3 glucan Derivative causes Regression of Solid Tumors in mice", BIOSCIENCE REPORTS, vol. 6, 1986, pages 845
- SELJELID R: "TUMOUR REGRESSION AFTER TREATMENT WITH AMINATED BETA1-3D POLYGLUCOSE IS INITIATED BY CIRCULATORY FAILURE", SCANDINAVIAN JOURNAL OF IMMUNOLOGY, BLACKWELL SCIENCE PUBL., OXFORD, GB, vol. 29, no. 2, 1 February 1989 (1989-02-01), pages 181 - 192, XP002071603, ISSN: 0300-9475
- CO TORELLO ET AL: "[beta]-1,3-Glucan given orally modulates immunomyelopoietic activity and enhances the resistance of tumour-bearing mice", CLINICAL AND EXPERIMENTAL PHARMACOLOGY AND PHYSIOLOGY, WILEY-BLACKWELL PUBLISHING ASIA, AU, vol. 39, no. 3, 22 February 2012 (2012-02-22), pages 209 - 217, XP071595105, ISSN: 0305-1870, DOI: 10.1111/J.1440-1681.2011.05655.X
- JIN YIMING ET AL: "[beta]-glucans as potential immunoadjuvants: A review on the adjuvanticity, structure-activity relationship and receptor recognition properties", VACCINE, vol. 36, no. 35, 1 August 2018 (2018-08-01), AMSTERDAM, NL, pages 5235 - 5244, XP055948377, ISSN: 0264-410X, DOI: 10.1016/j.vaccine.2018.07.038
- CHUNG YOUNG HUN ET AL: "Viral nanoparticles for drug delivery, imaging, immunotherapy, and theranostic applications", ADVANCED DRUG DELIVERY REVIEWS, ELSEVIER, AMSTERDAM , NL, vol. 156, 1 January 2020 (2020-01-01), pages 214 - 235, XP086393838, ISSN: 0169-409X, [retrieved on 20200627], DOI: 10.1016/J.ADDR.2020.06.024
- REIMERS MELISSA A ET AL: "Immunotherapy in Metastatic Castration-Resistant Prostate Cancer: Past and Future Strategies for Optimization", CURRENT UROLOGY REPORTS, CURRENT SCIENCE, US, vol. 20, no. 10, 3 September 2019 (2019-09-03), XP037918604, ISSN: 1527-2737, [retrieved on 20190903], DOI: 10.1007/S11934-019-0931-3
- SELJELID R ET AL: "Glycan stimulation of macrophages in vitro", EXPERIMENTAL CELL RESEARCH, ELSEVIER, AMSTERDAM, NL, vol. 131, no. 1, 1 January 1981 (1981-01-01), pages 121 - 129, XP024853880, ISSN: 0014-4827, [retrieved on 19810101], DOI: 10.1016/0014-4827(81)90413-4

## Description

### TECHNICAL FIELD

The present disclosure relates to a pharmaceutical combination. More specifically, the disclosure relates to a pharmaceutical combination for use in the treatment of cancer, in particular prostate cancer.

### BACKGROUND OF THE INVENTION

The concept of immune therapy of cancer is based on the extreme molecular precision of adaptive immune recognition, as well as the systems destructive capacity to annihilate or seriously damage the tumor tissue. The precision of adaptive immune recognition has been demonstrated repeatedly. On the other hand, the destructive capacity is generally questionable. This is probably the reason why immune therapy of cancer in general has not quite kept its promise.

For prostate cancer, there are no effective treatments, especially for advanced cancer of the prostate. Radical surgery is excluded when the cancer has penetrated outside the gland, which is often the case. Irradiation and chemotherapy are often of limited use because of serious side effects. In most cases, immunotherapy cannot be applied because of lack of tumor specific antigens in the tumor.

Recently, another venue opened when useful variants of oncolytic viruses were developed. Thus, a common approach today, is to combine oncolytic virus therapy and blocking antibodies to the programmed cell death-1 (PD-1) receptor in the treatment of prostate cancer however, with modest results in clinical trials (1).

Thus, despite strong efforts over the last decades to improve the treatment of cancer, there is still a need in the art to search for new pharmaceutical compositions or combinations for the treatment of cancer in general and prostate cancer in particular.

### SUMMARY OF THE INVENTION

It is an object of the present disclosure to mitigate, alleviate or eliminate one or more of the above-identified deficiencies and disadvantages in the prior art and solve at least the above-mentioned problem.

By the present invention, a novel effective treatment of prostate cancer, based on local activation of innate immunity has been provided. It has surprisingly been found that activation of the innate immune system in combination with well-known methods of treatment have superior effect in the treatment of prostate cancer.

Thus, according to a first aspect of the invention there is provided a pharmaceutical combination comprising an oncolytic virus immunotherapy, a programmed cell death-1 (PD-1) inhibitor and an activator of the innate immune system for use in the treatment of prostate cancer.

The oncolytic virus immunotherapy is talimogene laherparepvec. The PD-1 inhibitor is an anti-PD-1 antibody. The activator of the innate immune system is glucan, preferably β(1,3) glucan.

According to some embodiments, prostate cancer is advanced prostate cancer. Advanced prostate cancer is established outside the capsule of the prostate gland and is in most cases also metastatic.

According to some embodiments, the pharmaceutical combination for use in the treatment of prostate cancer is administered to a patient in need thereof in a therapeutically effective amount of the oncolytic virus, a therapeutically effective amount of the PD-1 inhibitor and a therapeutically effective amount of the activator of the innate immune system.

According to some embodiments, the therapeutically effective amount of glucan is at least about 1 gram per day, preferably at least 3 grams per day administered orally. No upper limit has been found. The activator of the innate immune system is administered every day during the treatment with oncolytic virus immunotherapy and the PD-1 inhibitor.

According to some embodiments, the oncolytic virus immunotherapy, PD-1 inhibitor and the innate immune system activator are administered in separate compositions.

According to some embodiments, the oncolytic virus immunotherapy, PD-1 inhibitor and the innate immune system activator are administered concurrently, or are administered at separate times or consecutively.

Preferably, the oncolytic virus immunotherapy is administered locally, the PD-1 inhibitor is administrated systemically, and the innate immune system activator is administrated orally.

It is herein described a method of treating prostate cancer in a human patient comprising the steps:
a) administering a therapeutically effective amount of an oncolytic virus,
b) administering of a therapeutically effective amount of the PD-1 inhibitor,
c) administering a therapeutically effective amount of the activator of the innate immune system.

Further disclosed is a pharmaceutical combination where the innate immune system activator is combined with a compound, compounds or a technology other than Tvec /PD-1 inhibitor to obtain a more effective treatment of cancer.

Examples of cancers to be treated are melanomas, lung cancer, mammary carcinoma and prostate cancer.

The present disclosure will become apparent from the detailed description given below. The detailed description and specific examples disclose preferred embodiments of the disclosure by way of illustration only. Those skilled in the art understand from guidance in the detailed description that changes and modifications may be made within the scope of the disclosure.

Hence, it is to be understood that the herein disclosed disclosure is not limited to the particular component parts of the device described or steps of the methods described since such device and method may vary. It is also to be understood that the terminology used herein is for the purpose of describing particular embodiments only, and is not intended to be limiting. It should be noted that, as used in the specification and the appended claim, the articles "a", "an", "the", and "said" are intended to mean that there are one or more of the elements unless the context explicitly dictates otherwise. Thus, for example, reference to "a unit" or "the unit" may include several devices, and the like. Furthermore, the words "comprising", "including", "containing" and similar wordings does not exclude other elements or steps.

In this context, the terms oncolytic virus immunotherapy, oncolytic virus agent, oncolytic virus and oncolytic agent are used interchangeably.

In this context, the term PD-1 (programmed cell death protein 1) inhibitor is to be understood to encompass inhibitors of PD-1 receptor and inhibitors of the ligand PD-L1 (programmed death-ligand). Thus, the terms PD-1 inhibitors and PD-L1 inhibitors are used interchangeably.

### BRIEF DESCRIPTIONS OF THE FIGURES

The above objects, as well as additional objects, features and advantages of the present disclosure, will be more fully appreciated by reference to the following illustrative and non-limiting detailed description of example embodiments of the present disclosure, when taken in conjunction with the accompanying drawings.
**Figure 1** shows mice with METH A tumor.
   Panel A shows the result on day 18 in mouse treated with one intravenous injection of aminated ß(1,3) glucan on day 7. No visible tumor on day 18.
   Panel B shows the result on day 18 in mouse treated with one intravenous injection of isotonic saline on day 7. Large tumor in the skin of abdomen.
**Figure 2** shows the results of treatment of human prostate cancer using the combination of the invention.
   Figure 2A shows the pronounced reduction of PSA, from 60 µg/l at the start of treatment to non-detectable amounts following 6 weeks of treatment.
   Figure 2B shows the pronounced reduction of LDH from 915 U/L to 173 U/L during the same time period.
   Figure 2 C is an MR picture taken prior to treatment showing progressive, vital growth of tumor tissue in all noted locations.
   Figure 2D is a MR picture taken after 9 weeks of treatment showing extensive necrosis of tumor tissue in all locations, i.e. os ileum, columna and elsewhere, also in soft tissues.

### DETAILED DESCRIPTION OF THE INVENTION

The present disclosure will now be described with reference to the accompanying drawings, in which preferred example embodiments of the disclosure are shown. The disclosure may, however, be embodied in other forms and should not be construed as limited to the herein disclosed embodiments. The disclosed embodiments are provided to fully convey the scope of the disclosure to the skilled person.

The concept of immune therapy of cancer is based on the extreme molecular precision of adaptive immune recognition, as well as the systems destructive capacity to annihilate or seriously damage the tumor tissue. The precision of adaptive immune recognition has been demonstrated repeatedly. On the other hand, the destructive capacity is generally questionable. This is probably the reason why clinical immune therapy of cancer in general has not quite kept its promise.

On the other hand, the destructive capacity of innate immunity is formidable, as demonstrated e.g. in certain cases of autoimmune disease.

It would seem to be of great promise for the immune therapy of cancer if one was able to combine the specificity of recognition of the adaptive system with the destructive capacity of an activated innate system. This is the scope of the present invention.

To be able to understand and accept the possible involvement of innate immunity in cancer therapy, a brief introduction to the complex matter of the two immune systems present in the vertebrates follows.

The fact that people as well as domestic animals having survived a certain infectious disease develops a degree of immunity to the same type of infection is probably Stone Age knowledge. Vaccination was invented in the 17th century. The underlying mechanism was believed to be stimulation to produce what we now call antibodies binding to the infection agent, increasing the absorption and killing in macrophages and leucocytes. The physiology of lymphocytes, T and B cells was not well understood before late in the 19th century.

The Russian biologist Elie Metchnikoff was awarded the Nobel Prize in 1908 for his work on macrophages and their important role in immunity (together with Paul Ehrlich, for Salvarsan). Metchnikoff studied a range of animal species and found that lower animals, invertebrates could not be vaccinated or similar, obviously could not produce antibodies but still had an excellent defense against infection. Therefore, there must be two types of immune systems.

We now know there are two main types, the adaptive system, only found in vertebrates (fish, amphibians, reptiles, birds and mammals). Invertebrates which constitute 95 % of all animals species do not have T and B cells, cannot produce antibodies, cannot mount an adaptive immune response, but do have macrophages and an excellent defense against infections, as good as vertebrates or maybe better. Vertebrates, mammals like us have innate immunity as well as adaptive immunity. Adaptive immune cells are organized in clones, which confers extreme specificity when the stimulated clones expand. The innate immune system is based on the reaction of vast numbers of identical or similar cells, i.e. macrophages and granulocytes, which results in rather poor specificity but very strong effect when the system is activated.

The early phase of acute inflammation in a healthy normal individual is a pure innate immune reaction. The reaction is triggered by a special tissue cell, i.e. the macrophage present in the tissues of all animals, also lower animals, invertebrates. When macrophages recognize foreign material or microbes, it immediately, i.e. within seconds or minutes start to produce substances that make small blood vessels leaky. Granulocytes normally only present inside the vessels, stream out to attack the foreign material, microbes or tissues. If the foreign matter, e.g. microbes, cannot be removed by the macrophages or granulocytes, the process may turn into a chronic inflammation, a complex process encompassing both innate and adaptive immunity components etc. Pure adaptive immunity is seen in successful vaccination, where antigen is injected intramuscularly or subcutaneously together with additional substances that will stimulate the adaptive reaction. These additional substances are called adjuvants. The difference between innate and adaptive immune reactions are striking. Innate immune response is fast, i.e. within seconds, minutes or hours. It is dramatic and effective, however rather unspecific. The adaptive immune response is slower and weaker, i.e. within weeks in nonimmune individual. However highly specific, and mature into a faster and stronger immune response in immunized individuals.

It has now surprisingly been shown that immune therapy taking advantage of the specificity of recognition of the adaptive system combined with the destructive capacity of an activated innate system are able to improve the treatment of prostate cancer. The specificity of the total reaction is provided by the adaptive immune system, that "tells" the innate part where the trouble is localized.

In particular, it has been shown that the effect of a combination of an oncolytic virus agent and PD-1 inhibitor is significantly improved when further combined with an activator of the innate immune system in the treatment of prostate cancer.

Thus, the present invention provides a pharmaceutical combination comprising an oncolytic virus agent, programmed cell death-1 (PD-1) inhibitor and an activator of the innate immune system for use in the treatment of prostate cancer. The pharmaceutical combination causes extensive destruction of tumor tissue.

### Oncolytic virus immunotherapy

The oncolytic virus immunotherapy comprises oncolytic viruses, which are viruses that infects and lyses cancer cells but not normal cells. It may be adenoviruses or herpes virus or other oncolytic viruses. In this context, any suitable oncolytic virus agent may be used. Preferably, the agent is an attenuated herpes simplex type 1-virus (HSV-1). More preferably, the agent is talimogene laherparepvec (Tvec).

Talimogene laherparepvec (Tvec) is an oncolytic virus commonly included in immunotherapy used to treat different cancer types, especially melanomas and prostate cancer. However, the effect of Tvec treatment alone, or in combination with a PD-1 inhibitor will only lead to modest effects in clinical trials.

The oncolytic virus agent included in the combination of the present invention is well known in the art and available as several approved pharmaceutical preparations administered according to the labels. Local administration is the most common.

### PD-1 inhibitor

Programmed cell death-1 (PD-1) is a cell surface receptor that inhibits the adaptive immune responses. It is a negative regulator of T-cell activity that has been shown to be involved in the control of T-cell immune responses. Inhibitors of the PD1 binds to the receptor and blocks its interaction with its ligands and counteract the inhibition of T cell activity.

PD-1 inhibitors and PD-L1 inhibitors are a group of checkpoint inhibitor anticancer drugs that block the activity of PD-1 and PD-L1 immune checkpoint proteins present on the surface of cells. Immune checkpoint inhibitors are emerging as a front-line treatment for several types of cancer.

The inhibitor of PD-1 may be any suitable PD-1 inhibitor. Preferably, the inhibitor of the PD-1 is an anti-PD-1 antibody. Examples of PD-1 inhibitors are pembrolizumab, ipilimumab nivolumab and cemiplimab. Examples of PD-L1 inhibitors are atezolizumab, avelumab and durvalumab. In this context, pembrolizumab is particularly preferred.

The PD-1 inhibitor included in the combination of the present invention is well known in the art and available as several approved pharmaceutical preparations administered according to the labels. Systemic administration is most common.

### Activators of the innate system

Activation of the innate immune system is based on the reaction of vast numbers of identical or similar cells, which results in rather poor specificity, but very strong reaction when the system is activated. Macrophages are ancient cells members of the innate immune system, found in varying forms all invertebrates that have been well studied.

The fight between macrophages and dangerous microbes, as well as fight between rivals early in phylogeny takes place in an environment almost devoid of protein. Almost all of extracellular macromolecules in primitive invertebrates are polysaccharides - glucans or glycans. Accordingly then, it would seem rational to look for activators of innate immune cells among natural polysaccharides - glycans or glucans.

In this context, the activator of the innate immune system may be any suitable activator of the innate immune system. Polysaccharides which are well-known activators of the innate immune system, particularly glycans and glucans.

More than forty polysaccharides were tested for their ability to stimulate macrophages *in vitro* (2) resulting in the following:
- All stimulatory polysaccharides (glycans or glucans) were either insoluble or gels. Water soluble natural glycans were not stimulatory.
- The stimulatory capacity of gel-like or insoluble glycans was correlated to the relative amount of ß(1,3) glucan (polyglucose) in the molecule.
- The strongest stimulatory glycans were pure ß(1,3) glucans.
- Not all insolubles were stimulatory such as for example chitin and cellulose.

The activator of the innate immune system is preferably glucan, more preferably β(1,3) glucan. The β(1,3) glucan is preferably in the form of a hydrophilic gel.

Glucans are a heterogeneous group of glucose polymers found in the cell walls of plants, bacteria, fungi and protozoa. Glucans have a backbone chain and in some cases side chains which, depending of the origin of the glucan, comprise β(1,3), β(1,4) and/or β(1,6)-linked glucosyl units. Depending upon the source and method of isolation, beta-glucans have various degrees of branching and type of linkage in the backbone and side chains. The frequency and type of linkage in the side chains is highly relevant to the molecule's biological activity. Glucans also differ highly in their molecular weight as well as in their tendency for chain aggregation, which both are essential features for the efficacy profile of these molecules. Most beta-glucans of fungal and yeast origin are in their native state insoluble in water, but can be made soluble either by acid hydrolysis or by derivatization introducing foreign groups like - phosphate, -sulphate, -amine, -carboxymethyl and so forth to the molecule. Some of these derivatives are stimulatory. Glucans made soluble by simple hydrolysis are not stimulatory.

The glucan used according to the present invention are disclosed in EP2646477 and available as the commercial product identified SBG (Soluble Beta-Glucan), a kind gift from Biotec Pharmacon, Tromsø, Norway in the form of an hydrophilic gel.

In preferred embodiments, the glucan is a β(1,3) glucan comprising a backbone of β(1,3)-linked glucosyl residues and side chains comprising 2 or more β(1,3)-linked glucosyl residues, the side chains being attached to the backbone via a β(1,6)-linkage.

Without being bound to any theory, the activation of the innate immune system may seem to be dependent of a helical structure of the activator molecule. A helical structure of glucan is attained when the glucan includes at least 5-6 β(1,3) glucosyl units.

The cancer types to be treated by the pharmaceutical combination of the invention may be any cancer type responding to the treatment. In principle, all cancers where traditional immune therapy has an observable, but insufficient effect should be eligible for additional treatment with an innate immune system activator. Examples are melanomas, both localized or advanced; lung cancer, mammary carcinoma and prostate cancer.

The cancer type to be treated by use of the pharmaceutical combination according to the invention is prostate cancer. Examples of prostate cancers are primary or localized prostate cancer, locally advanced prostate cancer, recurrent prostate cancer, advanced prostate cancer, metastatic prostate cancer, metastatic castration-resistant prostate cancer, and hormone- sensitive prostate cancer. In a preferred embodiment of the present invention, prostate cancer is advanced prostate cancer.

According to the present invention, the combination is administered to a patient in need thereof in a therapeutically effective amount of the oncolytic immunotherapeutic agent, a therapeutically effective amount of the PD-1 inhibitor and a therapeutically effective amount of the activator of the innate immune system. Said combination causes extensive destruction of tumor tissue.

It is herein described a method of treating prostate cancer in a human patient comprising the steps:
a) administering a therapeutically effective amount of an oncolytic virus agent,
b) administering a therapeutically effective amount of the PD-1 inhibitor,
c) administering a therapeutically effective amount of the activator of the innate immune system.

The oncolytic virus agent is talimogene laherparepvec, the PD-1 inhibitor is an anti-PD-1 antibody such as pembrolizumab and the activator of the innate immune system is β(1,3) glucan, preferably in form of a hydrophilic gel.

The oncolytic virus immunotherapy and the PD-1 inhibitor included in the combination of the present invention are well known in the art and available as several approved pharmaceutical preparations and administered according to the requirement and guidelines of the pharmaceutical labels. The activator of the innate immune system is administered every day during the treatment period with oncolytic virus agent and the PD-1 inhibitor. A typical period of treatment is about 8 weeks, wherein the oncolytic virus agent is administered locally followed by an intravenous injection of the PD-1 inhibitor the same day. The treatment is repeated with an interval of about two weeks. Typically, the treatment period is about 8 weeks. It may be beneficial to extend the administration of the activator of the innate immune system beyond the treatment period with oncolytic virus agent and the PD-1 inhibitor as no serious side effects are observed.

According to the present invention, the therapeutically effective amount of glucan is at least about 1 gram, preferably at least 3 grams per day administered orally. No upper limit has been found.

The oncolytic virus agent, PD-1 inhibitor and the innate immune system activator may be administered in separate compositions. They may be administered concurrently, or at separate times or consecutively.

The invention will now be further illustrated with reference to the following non-limiting examples.

### EXAMPLE 1

### Soluble glucan; aminated ß(1,3) glucan

Insoluble substances are unsuitable for *in vivo* experiments. Thus, efforts were made to provide soluble glucan derivatives.

Following advanced experiment on carbohydrate chemistry at the Institute of Wood Chemistry at the University of Uppsala, the inventor was able to provide a water soluble, stimulatory aminated β(1,3) glucan by treatment of natural, insoluble ß(1,3) with chloroacetaldehyde dimethyl acetal followed by reductive amination (3). The aminated ß(1,3) glucan derivative activated macrophages and thereby protected against otherwise lethal bacterial peritonitis in mice (4), as well as lethal virus infection in mice (not published).

### EXAMPLE 2

### Effect of treatment with ß(1,3) glucan in mice with implanted tumors

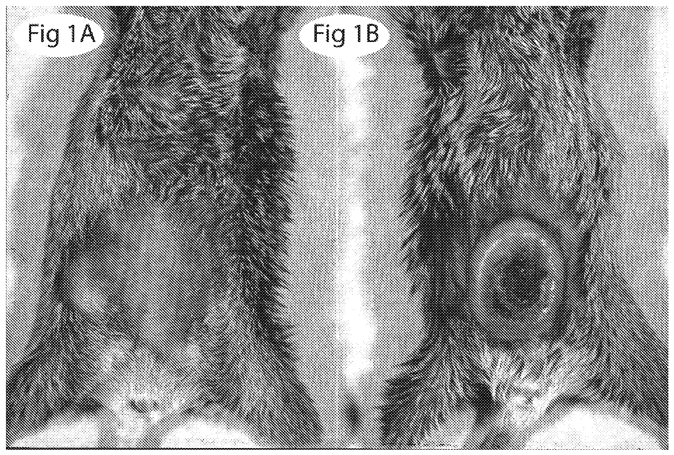

Meth A sarcoma was inoculated in the skin of hybrid CB6 F₁ (Balb/c x C 57131/6) and grew progressive in immunized animals. There was a characteristic period of slow growth around days 8-14 supposedly caused by a weak immune reaction after which the initial rapid growth resumed. When 5 mg of pure water-soluble aminated ß(1,3) glucan was injected intravenously or intraperitoneally on day 7, there was an almost immediate visible effect. (6,7) At 1 hour after the injection the tumor in mice treated with saline intravenously were reddish and bulging, (fig 1B) the tumors in mice having received ß(1,3) glucan intravenously appeared collapsed and whitish-grey. At 6 hours this was even more pronounced. Histological examination showed acute inflammation around the tumor in the glucan-treated animals with thickening of the walls of small vessels, stasis of red blood cells and perivascular collection of mononuclear cells (5). At day 18-21 after inoculation, tumor tissues in mice treated with ß(1,3) glucan were completely replaced by scar tissue Fig (1A). This was also the case after 200 days. The animals at this time point appeared to be unaffected and vital.

In TxB animals, (thymectomized, x-ray irradiated and bonemarrow transplanted) there was no effect of ß(1,3) glucan at day 8-14. ß(1,3) glucan treatment in non-immunized animals, or treated on day 1, 3 had little or no effect (5).

To decide whether the ß(1,3) glucan regression was peculiar to the Meth A tumor, we also performed experiments with sarcoma SA-1, syngeneic in A strain mice. The results were essentially identical to those documented with the Meth A, the only difference being that in about one third of the cases, tumors reappeared and resumed growth after 18-21 days.

As demonstrated, very small amounts of ß(1,3) glucan (µg) are sufficient to prevent a lethal outcome of a bacterial infection.

The result show that the effect of β(1,3) glucan is not a type of a new adjuvant, increasing the production of antibodies. Rather, the effect is an activation of the innate immunity by ß(1,3) glucan. An adjuvant effect, i.e. increased antibody production, is primarily seen after weeks. Cellular reaction, as in innate immune reactions, is seen after minutes or hours shown in the present study (5,6).

The aminated glucan is cumbersome to produce. We therefore tested the effect of non-derivatized, gel-like β(1,3) glucan produced by the firm Biotec Pharmacon (product named SBG- Soluble Beta Glucan), with essentially the same effect as with the aminated variety.

### EXAMPLE 3

### Treatment of prostate cancer by the combination of the invention

As noted in the introduction, immunotherapy of prostate cancer is difficult due to the lack of tumor specific antigens in the tumor. Oncolytic viruses - mediated therapy for prostate cancer has been tried with modest results (1). Recently, new therapies have developed combining oncolytic viruses with blocking antibodies such as pembrolizumab or similar, still with limited success at least for the treatment of prostate cancer.

The above study, shows the impressive effect of innate immune system activation. An obvious next step in the development of the application of innate immunity to cancer therapy would be to try it out in large groups of patients, especially since there are no hint of serious side-effects after intake of β(1,3) glucan per os.

So far, no clinical studies of this type are in progress. However, the present study is a report on one case study of a successful treatment based on modified immunization (Tvec and anti-PD-1, pembrolizumab), supported /followed by activation of innate immunity. The treatment was without significant side-effects, resulted in rapid normalization of prostate specific antigen (PSA) and lactate dehydrogenase (LDH), as well as extensive necrosis of tumor tissue as revealed by MR examination.

The following example shows the surprising synergistic effect of the combination of the invention, combining treatment with oncolytic viruses and anti-PD1 antibody with an activator of the innate immune system

The patient is 87 years old, generally in good health, active. Fell and had a compression fracture of Columna Th 11 in 2010, same year bypass surgery with excellent result, coxarthrosis bilaterally, protesis surgery left side 2005. Diagnosed with cancer of prostate autumn 2017, penetration of capsule towards vesicula seminalis, possible small metastasis os ileum right hand side, and possible small metastasis in columna L2, as revealed by MR examination. Histology: mostly Gleeson 5-7, in one spot Gleeson 9. PSA 10 µg/L at the time of diagnosis.

Received antihormon treatment (Zoladex) from May 2018 until present, radical x-ray treatment July- August 2018, Zometa from April 2018 to present, Xofigo March to April 2020. MR May 2020: progression of metastasis in os ileum with extensive soft tissue component, also progression in L2, new growth in L5 and right-hand femur diafysis.

During the first three months after diagnosis, the patient lost 10 kg of weight.

October 2020 palliative x-ray 8 Gy x 1 at os ileum and L2. Two small inguinal lymph nodes (approx. 2 x 1 cm) were noticed. The patient had moderate discomfort right hand iliac region from time of diagnosis.

The patient, which is a medical doctor himself, decided to try Tvec treatment and PD1 inhibition followed by activation of innate immunity with ß(1,3) polyglucose (glucan). Tvec was purchased from Amgen and PD1inhibitor pembrolizumab from MSD (Merck). SBG gel (soluble beta(1,3) glucan) was a gift from Biotec Pharmacon, Tromsø, Norway.

Tvec treatment was started on October 21, 2020 when 106 PFU was injected into the two left hand side inguinal lymph nodes, 200 mg pembrolizumab was given intravenously, and three grams of ß(1,3) glucan per os was given each day during the treatment period. On the following dates: November 11, December 2, and December 17, 108 PFU Tvec were injected into the inguinal lymph nodes and two hundred mg pembrolizumab was administered intravenously. Three grams of ß(1,3) glucan was given per os later each day. No side-effects were noticed, except for a slight flu-like condition a few hours following the Tvec injection. After the second round of parenteral medication a slight hypothyreosis developed. Except for these two observations, no significant side-effects were noticed.

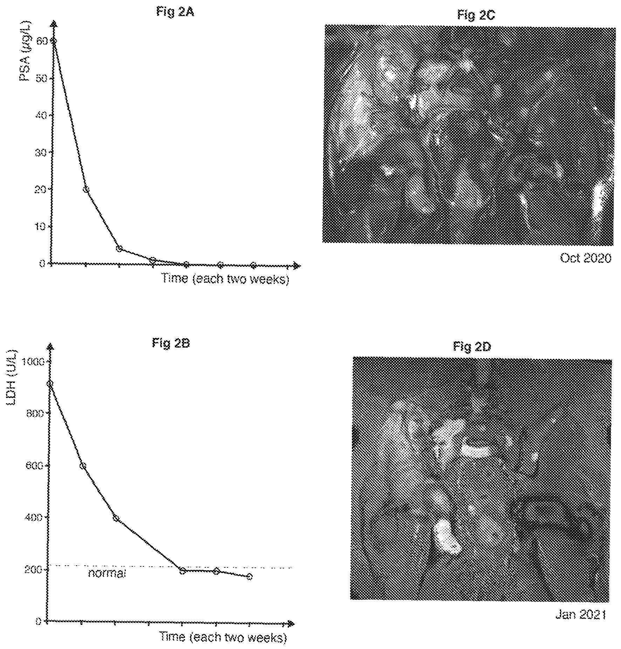

Pronounced reduction of PSA was recorded, from 60 µg/l at the start of treatment on October 21 to non-detectable amounts on December 17. (Fig. 2 A) During the same period, LDH was reduced from 915 to 173 U/L (fig.2 B). This situation persisted at least until the writing of this memo. MR on October 7th (before treatment) showed progressive, vital growth of tumor tissue in all noted locations. (Fig 2 C). MR on January 5 (after treatment) showed extensive necrosis of tumor tissue in all locations (os ileum, columna and elsewhere, also in soft tissues (Fig. 2 D). At the time of the termination of the treatment, the body weight loss has ceased. Presently, 8 months from start of treatment, the patient gains weight.

A twenty page expert description of de MR results was prepared (Signed: Wolfgang Picker, expert MR radiologist, not enclosed). The conclusion was as follows: "Strong regression of tumor components in the pelvis and no signs of increases in size of metastases outside of the pelvis. Tumor -cause fractures in pelvis and columna as before. No signs of emerging complications. Degenerative spinal stenosis as before".

Thus, it has surprisingly been provided a novel treatment for prostate cancer, based on the Tvec /pembrolizumab technology - supported by activation of innate immunity. The effect was striking. Reduction of PSA from 60 g/l to undetectable in 6 weeks, LDH from over 900 U/L to below 200 U/L (normal) during the same period, lasting at least as long as the total observation period. Extensive necrosis in the tumor tissue as revealed by MR. The treatment had no serious side-effects.

The above illustrates:
The fundamental underlying principle, in particular the destructive capacity and speed of reaction of the innate immune system.

The striking results from related preclinical investigations including mouse experiments with implanted tumors.

The potential of a novel, effective therapeutic procedure with little or no side-effects.

The speed of the reaction as seen in serum PSA and LDH concentrations, the duration of the effect (se fig 2 A and B) as well as the clear-cut and wide-spread MR change speaks for a following conclusion: we have found a way to combine the precision of the adaptive reaction with the effective destruction of tumor tissue by an activated innate immune reaction.

### Reference List

1. Katrina Sweeney & Gunnel Halldén Oncolytic Virotherapy 5: 45 2016 Oncolytic adenovirus-mediated therapy for prostate cancer.
2. Seljelid R, Bogwald J, Lundwall A, Exp. Cell Research 131:121, 1981 Glycan stimulation of macrophages in vitro.
3. Bogwald J, Hoffmann J, Seljelid R. Carbohydrate Res, 148, 101, 1986 Coupling of polysaccharides by means of chloroacetaldehyde dimethyl acetal to amines or protein by reductive amination.
4. Seljelid R, Bogvald J, Sjunneskog C, Hoffman J, Scand J. Immunol: 25: 50, 1987 A watersoluble aminated ß1-3 glucan derivative protects against E. coli infection in mice.
5. Seljelid R. Bioscience Reports 6: 845, 1986. A watersoluble aminated ß1-3 glucan Derivative causes Regression of Solid Tumors in mice.
6. R. Seljelid, Scand J. Immunology 29: 181, 1989 Tumour Regression after Treatment with aminated ß1-3 polyglucose is initiated by circulatory failure.

## Claims

1. Pharmaceutical combination comprising an oncolytic virus immunotherapy, a programmed cell death-1 (PD-1) inhibitor and an activator of the innate immune system for use in the treatment of prostate cancer, wherein the oncolytic virus immunotherapy is talimogene laherparepvec (Tvec), the PD-1 inhibitor is an anti-PD-1 receptor antibody and the activator of the innate immune system is β(1,3)glucan.

2. Pharmaceutical combination for use according to claim 1, wherein prostate cancer is advanced prostate cancer.

3. Pharmaceutical combination for use according to any of the preceding wherein the combination is administered to a patient in need thereof in a therapeutically effective amount of the oncolytic virus immunotherapy, a therapeutically effective amount of the PD-1 inhibitor and a therapeutically effective amount of the activator of the innate immune system.

4. Pharmaceutical combination for use according to claim 3, wherein the oncolytic virus immunotherapy is administered locally, the PD-1 inhibitor is administrated systemically, and the innate immune system activator is administrated orally.

5. Pharmaceutical combination for use according to any of the preceding claims, wherein the therapeutically effective amount of glucan is at least about 1 gram, preferably at least 3 grams per day administered orally.

6. Pharmaceutical combination for use according to any of the preceding claims wherein the oncolytic virus immunotherapy, the PD-1 inhibitor and the innate immune system activator are administered in separate compositions.

7. Pharmaceutical combination for use according to any of the preceding claims wherein the oncolytic virus immunotherapy, the PD-1 inhibitor and the innate immune system activator are administered concurrently or are administered at separate times or consecutively.

## Patentansprüche

1. Pharmazeutische Kombination, umfassend eine onkolytische Virusimmuntherapie, einen Inhibitor des Programmed Cell Death-1 (PD-1) und einen Aktivator des angeborenen Immunsystems, zur Verwendung bei der Behandlung von Prostatakrebs, wobei die onkolytische Virusimmuntherapie Talimogen laherparepvec (Tvec) ist, der PD-1-Inhibitor ein Anti-PD-1-Rezeptor-Antikörper ist und der Aktivator des angeborenen Immunsystems β(1,3)Glucan ist.

2. Pharmazeutische Kombination zur Verwendung nach Anspruch 1, wobei es sich bei dem Prostatakrebs um fortgeschrittenen Prostatakrebs handelt.

3. Pharmazeutische Kombination zur Verwendung nach einem der vorhergehenden Ansprüche, wobei die Kombination einem Patienten, der diese benötigt, in einer therapeutisch wirksamen Menge der onkolytischen Virusimmuntherapie, einer therapeutisch wirksamen Menge des PD-1-Inhibitors und einer therapeutisch wirksamen Menge des Aktivators des angeborenen Immunsystems verabreicht wird.

4. Pharmazeutische Kombination zur Verwendung nach Anspruch 3, wobei die onkolytische Virusimmuntherapie lokal verabreicht wird, der PD-1-Inhibitor systemisch verabreicht wird und der Aktivator des angeborenen Immunsystems oral verabreicht wird.

5. Pharmazeutische Kombination zur Verwendung nach einem der vorhergehenden Ansprüche, wobei die therapeutisch wirksame Menge an Glucan mindestens etwa 1 Gramm beträgt, vorzugsweise mindestens 3 Gramm pro Tag oral verabreicht.

6. Pharmazeutische Kombination zur Verwendung nach einem der vorhergehenden Ansprüche, wobei die onkolytische Virusimmuntherapie, der PD-1-Inhibitor und der Aktivator des angeborenen Immunsystems in getrennten Zusammensetzungen verabreicht werden.

7. Pharmazeutische Kombination zur Verwendung nach einem der vorhergehenden Ansprüche, wobei die onkolytische Virusimmuntherapie, der PD-1-Inhibitor und der Aktivator des angeborenen Immunsystems gleichzeitig verabreicht werden oder zu getrennten Zeitpunkten oder nacheinander verabreicht werden.

## Revendications

1. Combinaison pharmaceutique comprenant une immunothérapie par virus oncolytique, un inhibiteur de la mort cellulaire programmée-1 (PD-1) et un activateur du système immunitaire inné pour une utilisation dans le traitement du cancer de la prostate, dans laquelle l'immunothérapie par virus oncolytique est le talimogene laherparepvec (Tvec), l'inhibiteur de PD-1 est un anticorps anti-récepteur PD-1 et l'activateur du système immunitaire inné est le β(1,3)glucane.

2. Combinaison pharmaceutique pour une utilisation selon la revendication 1, dans laquelle le cancer de la prostate est un cancer de la prostate avancé.

3. Combinaison pharmaceutique pour une utilisation selon l'une quelconque des revendications précédentes, dans laquelle la combinaison est administrée à un patient en ayant besoin selon une quantité thérapeutiquement efficace de l'immunothérapie par virus oncolytique, une quantité thérapeutiquement efficace de l'inhibiteur de PD-1 et une quantité thérapeutiquement efficace de l'activateur du système immunitaire inné.

4. Combinaison pharmaceutique pour une utilisation selon la revendication 3, dans laquelle l'immunothérapie par virus oncolytique est administrée localement, l'inhibiteur de PD-1 est administré par voie systémique, et l'activateur du système immunitaire inné est administré par voie orale.

5. Combinaison pharmaceutique pour une utilisation selon l'une quelconque des revendications précédentes, dans laquelle la quantité thérapeutiquement efficace de glucane est d'au moins environ 1 gramme, de préférence d'au moins 3 grammes par jour administrée par voie orale.

6. Combinaison pharmaceutique pour une utilisation selon l'une quelconque des revendications précédentes, dans laquelle l'immunothérapie par virus oncolytique, l'inhibiteur de PD-1 et l'activateur du système immunitaire inné sont administrés dans des compositions distinctes.

7. Combinaison pharmaceutique pour une utilisation selon l'une quelconque des revendications précédentes, dans laquelle l'immunothérapie par virus oncolytique, l'inhibiteur de PD-1 et l'activateur du système immunitaire inné sont administrés simultanément ou sont administrés à des moments distincts ou consécutivement.
